(19) Europäisches Patentamt
European Patent Office
Office européen des brevets

(11) Veröffentlichungsnummer: **0 296 483 B1**

(12) **EUROPÄISCHE PATENTSCHRIFT**

(45) Veröffentlichungstag der Patentschrift: **06.05.92**

(51) Int. Cl.$^5$: **C07D 307/62**

(21) Anmeldenummer: **88109607.7**

(22) Anmeldetag: **16.06.88**

# (54) Herstellung von Ascorbinsäure-6-estern.

(30) Priorität: **26.06.87 CH 2431/87**
**24.03.88 CH 1112/88**

(43) Veröffentlichungstag der Anmeldung:
**28.12.88 Patentblatt 88/52**

(45) Bekanntmachung des Hinweises auf die Patenterteilung:
**06.05.92 Patentblatt 92/19**

(84) Benannte Vertragsstaaten:
**AT BE CH DE FR GB IT LI NL**

(56) Entgegenhaltungen:
**CH-A- 339 632**
**CH-A- 421 946**
**US-A- 3 250 790**

(73) Patentinhaber: **F. HOFFMANN-LA ROCHE AG**
**Postfach 3255**
**CH-4002 Basel(CH)**

(72) Erfinder: **Pauling, Horst, Dr.**
**Ruchholzstrasse 39**
**CH-4103 Bottmingen(CH)**
Erfinder: **Wehrli, Christof**
**Rheinstrasse 40**
**CH-4127 Birsfelden(CH)**

(74) Vertreter: **Urech, Peter, Dr. et al**
**Grenzacherstrasse 124 Postfach 3255**
**CH-4002 Basel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.18/2.0)

## Beschreibung

Die vorliegende Erfindung betrifft ein neues Verfahren zur Herstellung von Ascorbinsäure-6-estern, die wichtige alternative Anwendungsformen von L-Ascorbinsäure (Vitamin C) sind.

Vitamin C ist sowohl als Zusatz zu Nahrungsmitteln für Menschen und Tiere als auch als Antioxidans, insbesondere für Fette und Oele, bekannt. Die Vitamin C-Ester sind im Gegensatz zu Vitamin C selbst öllöslich, was beispielsweise zu einer erhöhten Wirksamkeit als Antioxidans führt.

Aufgabe der vorliegenden Erfindung ist die Schaffung eines Verfahrens, welches es ermöglicht, Ascorbinsäure-6-ester in einfacher und billiger Weise herzustellen, wobei der jeweils gewünschte Ascorbinsäure-6-ester in hoher Ausbeute und in guter Qualität anfällt. Das erfindungsgemässe Verfahren zur Herstellung von 6-aliphatischen $C_{2-20}$-Carbonsäureestern von Ascorbinsäure durch Veresterung der Ascorbinsäure mit einem aliphatischen $C_{2-20}$-Carbonsäurehalogenid ist dadurch gekennzeichnet, dass man die Veresterung durchführt in Gegenwart eines Halogenwasserstoffes sowie in Gegenwart eines N,N-Dialkyl-alkancarbonsäureamids der allgemeinen Formel

$R^1$-CONR$^2$R$^3$ I

oder eines cyclischen Amids der allgemeinen Formel

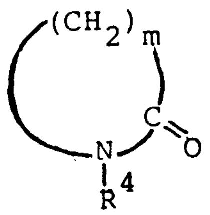

II

oder eines Tetraalkylcarbamids der allgemeinen Formel

$R^5R^6NCONR^7R^8$ III

oder eines cyclischen Carbamids der allgemeinen Formel

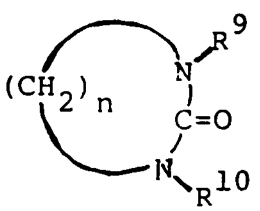

IV

worin $R^1$ geradkettiges oder verzweigtes $C_{1-5}$-Alkyl, $R^2$, $R^3$, $R^4$, $R^5$, $R^6$, $R^7$, $R^8$, $R^9$ und $R^{10}$ jeweils Methyl oder Aethyl, m 3, 4 oder 5 und n 2, 3 oder 4 bedeuten,
oder eines Phosphorsäuretriamids der allgemeinen Formel

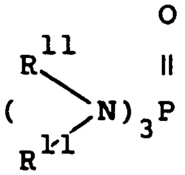

V

worin $R^{11}$ Methyl oder Aethyl bedeutet, oder die beiden Symbole $R^{11}$ zusammen mit dem Stickstoffatom, an das sie geknüpft sind, Piperidion bedeuten.

Der relevante Stand der Technik bezüglich Ascorbinsäureester ergibt folgendes Bild:

US-PS 3 250 790

**Ascorbinsäure + Fettsäure $\xrightarrow{BF_3}$ 6-Monoester**

Jap. Patentpubl. [62] 81307 (Mori)

Herstellung von Vitamin C-γ-linoleaten aus Linolensäurechloriden und Vitamin C möglich; ohne irgendwelche Angabe von Verfahrensdetails, weder für Monoester allgemein, noch für 6-Monoester im speziellen.

CH-PS 421 946
(US-PS 3318 914. DT-OS 1468 406) [Kobayashi]

Herstellung von 2,6-Diestern aus aliphatischen Carbonsäurehalogeniden in Gegenwart N,N-disubstituierter Amide unter basischen Bedingungen. Kolonne 2, Zeilen 35 seq. referieren ausgiebig über die Schwierigkeiten zur Herstellung von Monoestern.

CH-PS 339 632

Ascorbinsäure + ungesättigtes Fettsäurederivat --------> Gemisch von Monoestern

Unter "Ascorbinsäure" sind insbesondere die L-Ascorbinsäure und deren Enantiomeres, aber auch die D-Erythorbinsäure und deren Enantiomeres zu verstehen. Die 6-aliphatischen $C_{2-20}$-Carbonsäureester der Ascorbinsäure sowie die ebenfalls oben erwähnten aliphatischen $C_{2-20}$-Carbonsäurehalogenide weisen je nach Anzahl der Kohlenstoffatome eine gesättigte oder (einfach oder mehrfach) ungesättigte, geradkettige oder verzweigte aliphatische Acylgruppe auf. Beispiele von diesbezüglichen gesättigten, geradkettigen oder verzweigten Acylgruppen sind Acetyl, Propionyl, Butyryl, Pivaloyl, Decanoyl, Myristoyl, Palmitoyl und Stearoyl, während Oleoyl und Linoloyl Beispiele der ungesättigten Acylgruppen darstellen. Es kommen als aliphatische $C_{2-20}$-Carbonsäurehalogenide insbesondere die Säurechloride und Säurebromide in Frage.

Bevorzugte in dem erfindungsgemässen Verfahren verwendbare aliphatische $C_{2-20}$-Carbonsäurehalogenide sind das Palmitinsäurechlorid und das Linolsäurechlorid. Vorzugsweise wird unter Verwendung von diesen Säurechloriden die L-Ascorbinsäure erfindungsgemäss verestert.

Unter den erfindungsgemäss verwendbaren Substanzen (Lösungsmitteln), in deren Gegenwart die Veresterung durchgeführt wird, bilden die N,N-Dialkyl-alkancarbonsäureamide der Formel I, diejenigen cyclischen Amide der Formel II, worin $R^4$ Methyl bedeutet, Tetramethylharnstoff (Tetraalkylcarbamid der Formel III, in der $R^5$, $R^6$, $R^7$ und $R^8$ jeweils Methyl bedeuten), diejenigen cyclischen Carbamide der Formel IV, worin sowohl $R^9$ als auch $R^{10}$ Methyl bedeutet, und Hexamethylphosphorsäuretriamid (Phosphorsäuretriamid der Formel V, in der $R^{11}$ Methyl bedeutet) eine interessante Gruppe solcher Substanzen. Es sind N,N-Dimethylacetamid (Beispiel eines N,N-Dialkyl-alkancarbonsäureamids der Formel I); 1-Methyl-2-pyrrolidon (Beispiel eines cyclischen Amids der Formel II); Tetramethylharnstoff (Beispiel eines Tetraalkylcarbamids der Formel III); 1,3-Dimethyl-3,4,5,6-tetrahydro-2(1H)-pyrimidinon und 1,3-Dimethyl-2-imidazolidinon (Beispiele eines cyclischen Carbamids der Formel IV); und Hexamethylphosphorsäuretriamid (Beispiel eines Phosphorsäuretriamids der Formel V) bevorzugt, wobei N,N-Dimethylacetamid und 1-Methyl-2-pyrrolidon besonders bevorzugt sind.

Es hat sich als vorteilhaft erwiesen, die Ascorbinsäure in bis zu 25 gewichtsprozentigem Ueberschuss, insbesondere in ca. 10 gewichtsprozentigem Ueberschuss, bezogen auf die Menge Säurehalogenid, einzusetzen. Es wurde gefunden, dass die Verwendung überschüssiger Ascorbinsäure unerwünschte Mehrfachacylierung verhindert, z.B. die Bildung von 5,6-Ascorbyldipalmitat im Falle der Veresterung von L-Ascorbinsäure mit Palmitinsäurechlorid.

Im allgemeinen wird die Veresterung bei Temperaturen von -20 °C bis 40 °C durchgeführt, wobei die grössten Ausbeuten an Produkt bei Reaktionstemperaturen um 0 °C erzielt werden. Bei zu niedrigen Reaktionstemperaturen ist beispielsweise das Palmitinsäurechlorid zu wenig löslich, als dass eine einigermassen vollständige Veresterung erwartet werden kann. Bei zu hohen Temperaturen werden hingegen vermehrt Nebenprodukte gebildet.

Die Ascorbinsäure ist je nach erfindungsgemäss verwendetem Lösungsmittel in diesem bis zu ca. 35% löslich, wobei die Lösungen hoher Konzentration unerwünscht viskos sind. Es hat sich gezeigt, dass man zur Verringerung der Viskosität des Reaktionsgemisches ein zusätzliches inertes Lösungsmittel verwenden kann, ohne dass der Verlauf der Veresterung beeinträchtigt wird. Die Gegenwart dieses zusätzlichen

Lösungsmittels wirkt auf die Veresterung sogar vorteilhaft, da dadurch eine höhere Ausbeute an Produkt erzielt werden kann. Bevorzugte zusätzliche inerte Lösungsmittel sind halogenierte (insbesondere chlorierte) aliphatische Kohlenwasserstoffe, insbesondere Methylenchlorid und Chloroform; cyclische Aether, insbesondere Tetrahydrofuran und Dioxan, sowie Acetonitril. Bei Verwendung des zusätzlichen inerten Lösungsmittels beträgt das Volumenverhältnis der erfindungsgemässen verwendeten Substanz (Lösungsmittel) zum inerten Lösungsmittel vorzugsweise 1:0,1 bis 1:1, insbesondere ca. 1:0,5.

Es wurde gefunden, dass durch Zugabe eines Halogenwasserstoffes, insbesondere von Chlorwasserstoff, zum Reaktionsgemisch die Ausbeute an Produkt wesentlich gesteigert wird, und zwar insbesondere bei Verwendung von N,N-Dimethylacetamid, 1-Methyl-2-pyrrolidon, Tetramethylharnstoff, 1,3-Dimethyl-3,4,5,6-tetrahydro-2(1H)-pyrimidinon oder 1,3-Dimethyl-2-imidazolidinon als (Haupt)Lösungsmittel. Bei steigendem Zusatz von Halogenwasserstoff bis zu einem Molverhältnis Ascorbinsäure zum Halogenwasserstoff von 1:2, insbesondere im Bereich von 1:0,1 bis 1:2, werden zunehmende Ausbeuten an Produkt erzielt. Dieses Verhältnis beträgt bei einer besonders bevorzugten Ausführungsform etwa 1:1.

Die erfindungsgemäss hergestellten Ascorbinsäure-6-ester können nach an sich bekannten Methoden isoliert und gereinigt werden. Vorzugsweise wird beispielsweise im Falle der Herstellung von 6-Ascorbylpalmitat das Reaktionsgemisch nach Beendigung der Veresterung mit Wasser sowie einem geeigneten organischen Lösungsmittel, insbesondere Diäthyläther, Diisopropyläther, Essigester oder Isobutylmethylketon, verdünnt und das resultierende 6-Ascorbylpalmitat in kristalliner Form vom verdünnten Reaktionsgemisch abfiltriert. Nach dem Abfiltrieren und anschliessendem Trocknen liegt das so isolierte 6-Ascorbylpalmitat normalerweise als weisses Kristallisat vor, das keiner weiteren Reinigung bedarf, da allfällige Nebenprodukte, z.B. kleinere Mengen 5-Ascorbylpalmitat und 5,6-Ascorbyldipalmitat, im Filtrat zurückbleiben. Aus dem wässrigen Filtrat kann das bei der Veresterung verwendete Lösungsmittel, z.B. Dimethylacetamid, auf konventionelle Weise, z.B. Destillation oder Extraktion, in guter Ausbeute zurückgewonnen und rezyklisiert werden, was einen weiteren Vorteil des erfindungsgemässen Verfahrens darstellt.

Im Gegensatz zu dem 6-Ascorbylpalmitat wird beispielsweise das 6-Ascorbyllinolat in nichtkristalliner Form hergestellt. Die meisten polaren und unpolaren Verunreinigungen sowie Nebenprodukte können mittels Verteilungsextraktion entfernt werden. Wenn das erfindungsgemäss verwendete Linolsäurehalogenid, was normalerweise der Fall ist, aus einer Linolsäure technischer Qualität deriviert wird, welche aus einem Gemisch von ca. 60% Linolsäure, 30% Oelsäure sowie weiteren ungesättigten Fettsäuren besteht, besteht das damit hergestellte 6-Ascorbyllinolat ebenfalls aus einem Produktgemisch, welches die Zusammensetzung der eingesetzten Linolsäure wiederspiegelt. Bei Verwendung reiner Linolsäure erhält man dementsprechend reines Linolat.

Das erfindungsgemäss hergestellte 6-Ascorbyllinolat ist neu und bildet einen weiteren Gegenstand der vorliegenden Erfindung. Dank seiner überraschend hohen Fettlöslichkeit ist das 6-Ascorbyllinolat ein besonders interessantes Antioxidans, das sich beispielsweise zur antioxidativen Stabilisierung von Pflanzenölen gut eignet.

Das erfindungsgemässe Verfahren wird durch die nachfolgenden Beispiele weiter veranschaulicht.

## Beispiel 1

### Herstellung von 6-Ascorbylpalmitat

In einem mit Thermometer, Gaseinleitungsrohr, Rührer sowie Tropftrichter versehenen Reaktionsgefäss werden bei 0 °C 26,5 ml N,N-Dimethylacetamid vorgelegt. Dann werden bei 10 °C 2,0 g (55 mMol) Chlorwasserstoffgas eingeleitet und 9,69 g (55 mMol) kristalline L-Ascorbinsäure sowie 13,25 ml Methylenchlorid zugegeben. Auf die resultierende klare Lösung werden bei 0 °C innert 4 Stunden 15,2 ml (50 mMol) Palmitinsäurechlorid zugetropft. Das Reaktionsgemisch wird 18 Stunden bei 0 °C und anschliessend 30 Minuten bei 20 °C gerührt.

Zum Reaktionsgemisch werden nun 100 ml Essigester (als Alternative kann hier u.a. Diäthyläther, Diisopropyläther oder Isobutylmethylketon verwendet werden) sowie 200 ml Wasser gegeben. Das wässrige Gemisch wird 2 Stunden bei 0 °C gerührt. Anschliessend nutscht man bei 0 °C das auskristallisierte Produkt ab, teigt den Filterkuchen dreimal mit je 50 ml Wasser gut an und wäscht ihn nach. Schliesslich wird das gut trocken gesaugte Kristallisat 18 Stunden am Wasserstrahlvakuum bei 50 °C bis zur Gewichtskonstanz getrocknet.

Auf diese Weise erhält man das 6-Ascorbylpalmitat (17,97 g, was einer Ausbeute von 86,7% der theoretischen entspricht), Smp. 112-113 °C; $[\alpha]_D^{20}$ + 21,1 (1% Aethanol); Reinheit nach Hochdruckflüssigchromatographie 99%.

Beispiel 2

Das im Beispiel 1 beschriebene Verfahren wird analog durchgeführt, indem anstelle von N,N-Dimethylacetamid N-Methyl-2-pyrrolidon als Lösungsmittel verwendet wird. Auf diese Weise erhält man 17,30 g 6-Ascorbylpalmitat (83,5% der theoretischen Ausbeute), Smp. 112-113° C; $[\alpha]_D^{20}$ = +21,3 (1% Aethanol); Reinheit nach Hochdruckflüssigchromatographie 99%.

Beispiel 3

Das im Beispiel 1 beschriebene Verfahren wird analog durchgeführt, indem anstelle von N,N-Dimethylacetamid und Methylenchlorid Hexamethylphosphorsäuretriamid resp. Tetrahydrofuran als Lösungsmittel verwendet werden. Auf diese Weise erhält man 17,80 g 6-Ascorbylpalmitat (86,0% der theoretischen Ausbeute), Smp. 112-113° C; Reinheit nach Hochdruckflüssigchromatographie 99%.

Beispiel 4

Herstellung von 6-Ascorbyllinolat

In einem mit Thermometer, Magnetrührer, Tropftrichter, Rückflusskühler sowie Gaswaschflaschen versehenen Reaktionsgefäss werden unter Schutzgas 59,2 g (211 mMol) Linolsäure (technische Qualität: bestehend aus ca. 55% Linolsäure, 35% Oelsäure sowie weiteren ungesättigten Fettsäuren), 60 ml Toluol und 17 ml (234 mMol) Thionylchlorid vereinigt. Zum Gemisch wird als Katalysator 0,5 ml Dimethylformamid gegeben, und das Ganze wird dann ca. 18 Stunden bei Raumtemperatur gerührt, und zwar bis zur Beendung der Gasentwicklung (Schwefeldioxid, Chlorwasserstoff). Zwecks Entfernen vom überschüssigen Thionylchlorid wird die resultierende Lösung am Rollverdampfer am Wasserstrahlvakuum bei 45° C eingeengt. Als Rückstand verbleiben 63,1 g Linolsäurechlorid.

In einem mit Thermometer, Gaseinleitungsrohr sowie Rührer versehenen Reaktionsgefäss werden unter Schutzgas 120 ml N,N-Dimethylacetamid vorgelegt. Dann werden bei 10° C 8,8 g (240 mMol) Chlorwasserstoffgas eingeleitet und 42,3 g (240 mMol) kristalline L-Ascorbinsäure sowie 60 ml Methylenchlorid zugegeben. Auf die resultierende klare Lösung werden bei 0° C (Eisbadtemperatur) innert 4 Stunden 72.6 g (211 mMol) Linolsäurechlorid zugetropft. Das Reaktionsgemisch wird 18 Stunden bei 0° C gerührt.

Zum Reaktionsgemisch werden nun 400 ml Wasser sowie 400 ml Diäthyläther gegeben. Das wässrige Gemisch wird 10 Minuten gerührt und anschliessend dreimal mit je 100 ml Diäthyläther extrahiert. Die organischen Phasen werden vereinigt und über wasserfreiem Natriumsulfat getrocknet. Die Lösung wird am Rollverdampfer am Wasserstrahlvakuum bei 40° C eingeengt. Schliesslich wird der resultierende feste Rückstand 18 Stunden bei 30° C am Wasserstrahlvakuum über Phosphorpentoxid bis zur Gewichtskonstanz getrocknet. Auf diese Weise erhält man das 6-Ascorbyllinolat (86,6 g, was einer Ausbeute von 93,6% der theoretischen entspricht) als festes Harz, $[\alpha]_D^{20}$ + 17,4 (1% Aethanol).

## Patentansprüche

1. Verfahren zur Herstellung von 6-aliphatischen $C_{2-20}$-Carbonsäureestern von Ascorbinsäure durch Veresterung der Ascorbinsäure mit einem aliphatischen $C_{2-20}$-Carbonsäurehalogenid, dadurch gekennzeichnet, dass man die Veresterung durchführt in Gegenwart eines Halogenwasserstoffes sowie in Gegenwart eines N,N-Dialkyl-alkancarbonsäureamids der allgemeinen Formel

   $R^1$-CONR$^2$R$^3$ I

   oder eines cyclischen Amids der allgemeinen Formel

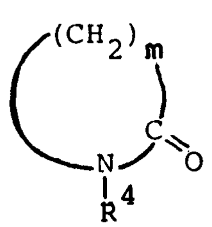

II

oder eines Tetraalkylcarbamids der allgemeinen Formel

$R^5R^6NCONR^7R^8$ III

oder eines cyclischen Carbamids der allgemeinen Formel

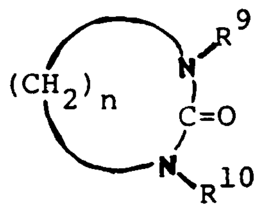

IV

worin $R^1$ geradkettiges oder verzweigtes $C_{1-5}$-Alkyl, $R^2$, $R^3$, $R^4$, $R^5$, $R^6$, $R^7$, $R^8$, $R^9$ und $R^{10}$ jeweils Methyl oder Aethyl, m 3, 4 oder 5 und n 2, 3 oder 4 bedeuten,
oder eines Phosphorsäuretriamids der allgemeinen Formel

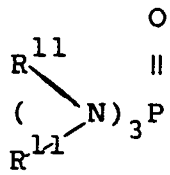

V

worin $R^{11}$ Methyl oder Aethyl bedeutet, oder die beiden Symbole $R^{11}$ zusammen mit dem Stickstoffatom, an das sie geknüpft sind, Piperidino bedeuten.

**2.** Verfahren nach Anspruch 1, dadurch gekennzeichnet, dass man die Veresterung in Gegenwart eines N,N-Dialkyl-alkancarbonsäureamids der Formel I oder eines cyclischen Amids der Formel II, in der $R^4$ Methyl bedeutet, oder von Tetramethylharnstoff oder eines cyclischen Carbamids der Formel IV, in der sowohl $R^9$ als auch $R^{10}$ Methyl bedeutet, oder von Hexamethylphosphorsäuretriamid durchführt.

**3.** Verfahren nach Anspruch 1 oder 2, dadurch gekennzeichnet, dass man die L-Ascorbinsäure verestert.

**4.** Verfahren nach einem der Ansprüche 1 bis 3, dadurch gekennzeichnet, dass man als aliphatisches $C_{2-20}$-Carbonsäurehalogenid das Palmitinsäurechlorid oder das Linolsäurechlorid verwendet.

**5.** Verfahren nach einem der Ansprüche 1 bis 4, dadurch gekennzeichnet, dass man die Veresterung in Gegenwart von N,N-Dimethylacetamid, 1-Methyl-2-pyrrolidon, Tetramethylharnstoff, Hexamethylphosphorsäuretriamid, 1,3-Dimethyl-3,4,5,6-tetrahydro-2(1H)-pyrimidinon oder 1,3-Dimethyl-2-imidazolidinon durchführt.

**6.** Verfahren nach Anspruch 5, dadurch gekennzeichnet, dass man in Gegenwart von N,N-Dimethylacetamid oder 1-Methyl-2-pyrrolidon verestert.

**7.** Verfahren nach einem der Ansprüche 1 bis 6, dadurch gekennzeichnet, dass die Ascorbinsäure in bis zu 25 gewichtsprozentigem Ueberschuss eingesetzt wird.

**8.** Verfahren nach einem der Ansprüche 1 bis 7, dadurch gekennzeichnet, dass die Veresterung bei Temperaturen von -20 °C bis 40 °C durchgeführt wird.

**9.** Verfahren nach einem der Ansprüche 1 bis 8, dadurch gekennzeichnet, dass man zur Verringerung der Viskosität des Reaktionsgemisches ein inertes Lösungsmittel zusetzt.

**10.** Verfahren nach Anspruch 9, dadurch gekennzeichnet, dass das inerte Lösungsmittel Methylenchlorid, Chloroform, Tetrahydrofuran, Dioxan oder Acetonitril ist.

**11.** Verfahren nach Anspruch 9 oder 10, dadurch gekennzeichnet, dass das Volumenverhältnis N,N-Dialkylalkancarbonsäureamid der Formel I, cyclisches Amid der Formel II, Tetraalkylcarbamid der Formel III, cyclisches Carbamid der Formel IV oder Phosphorsäuretriamid der Formel V zum inerten Lösungsmittel von 1:0,1 bis 1:1 beträgt.

**12.** Verfahren nach einem der Ansprüche 1 bis 11, dadurch gekennzeichnet, dass das Molverhältnis Ascorbinsäure zum Halogenwasserstoff von 1:0,1 bis 1:2 beträgt.

**13.** Verfahren nach Anspruch 12, dadurch gekennzeichnet, dass der Halogenwasserstoff Chlorwasserstoff ist.

**14.** Verfahren nach einem der Ansprüche 3 bis 13, dadurch gekennzeichnet, dass man das erhaltene 6-Ascorbylpalmitat isoliert, indem man das Reaktionsgemisch mit Wasser und Diäthyläther, Diisopropyläther, Essigester oder Isobutylmethylketon verdünnt und das resultierende 6-Ascorbylpalmitat in kristalliner Form vom verdünnten Reaktionsgemisch abfiltriert.

**Claims**

**1.** A process for the manufacture of 6-aliphatic $C_{2-20}$-carboxylic acid esters of ascorbic acid by esterifying the ascorbic acid with an aliphatic $C_{2-20}$-carboxylic acid halide, characterized by carrying out the esterification in the presence of a hydrogen halide and in the presence of a N,N-dialkylalkanecarboxylic acid amide of the general formula

$R^1$-$CONR^2R^3$ I

or of a cyclic amide of the general formula

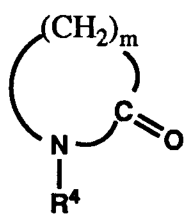

II

or of a tetraalkylcarbamide of the general formula

$R^5R^6NCONR^7R^8$ III

or of a cyclic carbamide of the general formula

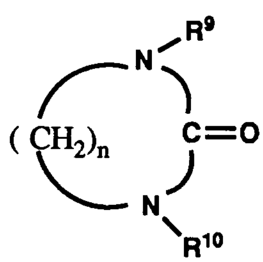

IV

wherein $R^1$ signifies straight-chain or branched $C_{1-5}$-alkyl, $R^2$, $R^3$, $R^4$, $R^5$, $R^6$, $R^7$, $R^8$, $R^9$ and $R^{10}$ each signify methyl or ethyl, m signifies 3, 4 or 5 and n signifies 2, 3 or 4,
or of a phosphoric acid triamide of the general formula

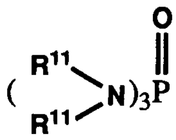

V

wherein $R^{11}$ signifies methyl or ethyl or the two symbols $R^{11}$ together with the nitrogen atom to which they are attached signify piperidino.

**2.** A process according to claim 1, characterized in that esterification is carried out in the presence of a N,N-dialkyl-alkanecarboxylic acid amide of formula I or of a cyclic amide of formula II in which $R^4$ signifies methyl or of tetramethylurea or of a cyclic carbamide of formula IV in which not only $R^9$ but also $R^{10}$ signifies methyl or of hexamethylphosphoric acid triamide.

**3.** A process according to claim 1 or 2, characterized in that L-ascorbic acid is esterified.

**4.** A process according to any one of claims 1 to 3, characterized in that palmitoyl chloride or linoloyl chloride is used as the aliphatic $C_{2-20}$-carboxylic acid halide.

**5.** A process according to any one of claims 1 to 4, characterized in that the esterification is carried out in the presence of N,N-dimethylacetamide, 1-methyl-2-pyrrolidone, tetramethylurea, hexamethylphosphoric acid triamide, 1,3-dimethyl-3,4,5,6-tetrahydro-2(1H)-pyrimidinone or 1,3-dimethyl-2-imidazolidinone.

**6.** A process according to claim 5, characterized in that the esterification is carried out in the presence of N,N-dimethylacetamide or 1-methyl-2-pyrrolidone.

**7.** A process according to any one of claims 1 to 6, characterized in that the ascorbic acid is used in up to 25% weight percent excess.

**8.** A process according to any one of claims 1 to 7, characterized in that the esterification is carried out at temperatures of -20 °C to 40 °C.

**9.** A process according to any one of claims 1 to 8, characterized in that an inert solvent is added to decrease the viscosity of the reaction mixture.

**10.** A process according to claim 9, characterized in that the inert solvent is methylene chloride, chloroform, tetrahydrofuran, dioxan or acetonitrile.

**11.** A process according to claim 9 or 10, characterized in that the volume ration of N,N-dialkyl-alkanecarboxylic acid amide of formula I, cyclic amide of formula II, tetraalkylcarbamide of formula III, cyclic carbamide of formula IV or phosphoric acid triamide of formula V to the inert solvent is from 1:0.1 to 1:1.

**12.** A process according to any one of claims 1 to 11, characterized in that the molar ratio of ascorbic acid to hydrogen halide is from 1:0.1 to 1:2.

**13.** A process according to claim 12, characterized in that the hydrogen halide is hydrogen chloride.

**14.** A process according to any one of claims 3 to 13, characterized in that the 6-ascorbyl palmitate obtained is isolated by diluting the reaction mixture with water and diethyl ether, diisopropyl ether, ethyl acetate or isobutyl methyl ketone and filtering off the resulting 6-ascorbyl palmitate in crystalline form from the diluted reaction mixture.

## Revendications

**1.** Procédé de préparation d'esters aliphatiques d'acides carboxyliques en $C_2$ à $C_{20}$ , en 6 de l'acide ascorbique par estérification de l'acide ascorbique avec un halogénure d'acide carboxylique en $C_2$ à $C_{20}$ aliphatique, caractérisé en ce qu'on conduit l'estérification en présence d'un acide halohydrique ainsi que d'un amide d'acide N,N-dialcoyl-alcanecarboxylique de formule générale

$R^1$-CONR$^2$R$^3$ I

ou d'un amide cyclique de formule générale

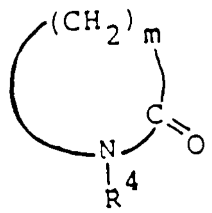

II

ou d'un tétraalcoylcarbamide de formule générale

$R^5R^6NCONR^7R^8$ III

ou d'un carbamide cyclique de formule générale

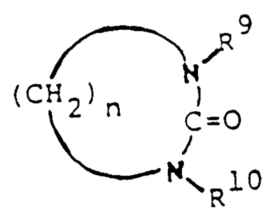

IV

dans laquelle $R^1$ représente un alcoyle en $C_1$ à $C_5$ à chaîne droite ou ramifiée, $R^2$, $R^3$, $R^4$, $R^5$, $R^6$, $R^7$, $R^8$, $R^9$ et $R^{10}$ représentent chacun un méthyle ou un éthyle, m vaut 3, 4 ou 5 et n vaut 2, 3 ou 4,
ou d'un triamide de l'acide phosphorique de formule générale

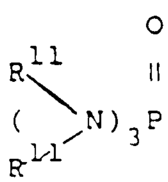

V

dans laquelle $R^{11}$ représente un méthyle ou un éthyle, ou les deux symboles $R^{11}$ représentent, avec l'atome d'azote auquel ils sont liés, un pipéridino.

**2.** Procédé selon la revendication 1, caractérisé en ce qu'on conduit l'estérification en présence d'un amide d'acide N,N-dialcoylalcanecarboxylique de formule I ou d'un amide cyclique de formule II, dans laquelle $R^4$ représente un méthyle, ou de tétraméthylurée ou d'un carbamide cyclique de formule IV dans laquelle tant $R^9$ que $R^{10}$ représentent un méthyle, ou de triamide de l'acide hexaméthylphosphorique.

**3.** Procédé selon la revendication 1 ou 2, caractérisé en ce qu'on estérifie l'acide L-ascorbique.

4. Procédé selon l'une des revendications 1 à 3, caractérisé en ce qu'on utilise comme halogénure d'acide carboxylique en $C_2$ à $C_{20}$ aliphatique le chlorure de l'acide palmitique ou le chlorure de l'acide linoléique.

5. Procédé selon l'une des revendications 1 à 4, caractérisé en ce qu on conduit l'estérification en présence de N-N-diméthylacétamide, de 1-méthyl-2-pyrrolidone, de tétraméthylurée, de triamide de l'acide hexaméthylphosphorique, de 1,3-diméthyl-3,4,5,6-tétrahydro-2(1H)-pyrimidinone ou de 1,3-diméthyl-2-imidazolidinone.

6. Procédé selon la revendication 5, caractérisé en ce qu'on estérifie en présence de N,N-diméthylacétamide ou de 1-méthyl-2-pyrrolidone.

7. Procédé selon l'une des revendications 1 à 6, caractérisé en ce qu'on utilise l'acide ascorbique en un excès allant jusqu'à 25% en poids.

8. Procédé selon l'une des revendications 1 à 7, caractérisé en ce qu on conduit l'estérification à des températures allant de -20 °C à 40 °C.

9. Procédé selon l'une des revendications 1 à 8, caractérisé en ce que pour diminuer la viscosité du mélange réactionnel on ajoute un solvant inerte.

10. Procédé selon la revendication 9, caractérisé en ce que le solvant inerte est le chlorure de méthylène, le chloroforme, le tétrahydrofuranne, le dioxanne ou l'acétonitrile.

11. Procédé selon la revendication 9 ou 10, caractérisé en ce que le rapport volumique de l'amide de l'acide N,N-dialcoyl-alcanecarboxylique de formule I, de l'amide cyclique de formule II, du tétraalcoylcarbamide de formule III, du carbamide cyclique de formule IV ou du triamide de l'acide phosphorique de formule V au solvant inerte s'élève de 1:0,1 à 1:1.

12. Procédé selon l'une des revendications 1 à 11, caractérisé en ce que le rapport molaire de l'acide ascorbique à l'acide halohydrique s'élève de 1:0,1 à 1:2.

13. Procédé selon la revendication 12, caractérisé en ce que l'acide halohydrique est l'acide chlorhydrique.

14. Procédé selon l'une des revendications 3 à 13, caractérisé en ce qu'on isole le 6-ascorbylpalmitate obtenu en diluant le mélange réactionnel obtenu avec de l'eau et du diéthyléther, du diisopropyléther, de l'acétate d'éthyle ou de l'isobutylméthylcétone et en ce qu'on sépare du mélange réactionnel par filtration le 6-ascorbylpalmitate résultant sous forme cristalline.